# EUROPÄISCHE PATENTSCHRIFT

(11) **EP 3 164 066 B1**
(45) Veröffentlichungstag und Bekanntmachung des Hinweises auf die Patenterteilung: **08.05.2019**
(21) Anmeldenummer: 15738266.4
(22) Anmeldetag: 01.07.2015
(51) Int. Cl.: A61B 5/053, H01R 13/66

(54) **SYSTEM FÜR EINEN IMPEDANZTOMOGRAPHEN**
SYSTEM FOR AN IMPEDANCE TOMOGRAPH
SYSTÈME POUR UN TOMOGRAPHE PAR IMPÉDANCE

(30) Priorität: 04.07.2014 DE 102014009889
(43) Veröffentlichungstag der Anmeldung: 10.05.2017
(73) Patentinhaber: Drägerwerk AG & Co. KGaA, 23558 Lübeck (DE)
(72) Erfinder: SATTLER, Frank, 23560 Lübeck (DE); HILTAWSKY, Karsten, 23617 Stockelsdorf (DE); LI, Jian Hua, 23558 Lübeck (DE)
(74) Vertreter: Mildner, Volker
(86) Internationale Anmeldenummer: PCT/EP2015/001321
(87) Internationale Veröffentlichungsnummer: WO 2016/000822

(56) Entgegenhaltungen:
- DE-A1-102005 041 385
- US-A- 4 744 369
- US-A1- 2002 079 910
- US-A1- 2013 345 535
- WILLIAM R B LIONHEART ET AL: "Generalized optimal current patterns and electrical safety in EIT; Generalized optimal current patterns and electrical safety in EIT", PHYSIOLOGICAL MEASUREMENT, INSTITUTE OF PHYSICS PUBLISHING, BRISTOL, GB, Bd. 22, Nr. 1, 1. Februar 2001 (2001-02-01), Seiten 85-90, XP020073514, ISSN: 0967-3334, DOI: 10.1088/0967-3334/22/1/311

## Beschreibung

Die Erfindung betrifft ein System mit einer Verbindungseinheit mit deren Hilfe ein Impedanztomograph mit einer ersten und einer zweiten Hautelektroden eines Elektrodenträgers des erfindungsgemäßen Systems elektrisch verbindbar ist, wobei die Hautelektroden in Längsrichtung des Elektrodenträgers voneinander beabstandet an dem Elektrodenträger angeordnet sind, eine erste Gegenkontaktvorrichtung an dem Elektrodenträger angeordnet und mit der ersten Hautelektrode verbunden ist, eine zweiten Gegenkontaktvorrichtung an dem Elektrodenträger angeordnet und mit der zweiten Hautelektrode verbunden ist, die Verbindungseinheit eine erste und eine zweite Kontaktvorrichtung aufweist, die erste Kontaktvorrichtung mit der ersten Gegenkontaktvorrichtung lösbar verbindbar ausgebildet ist, und die zweite Kontaktvorrichtung mit der zweiten Gegenkontaktvorrichtung lösbar verbindbar ausgebildet ist.

Elektrodiagnostische Verfahren werden häufig an Patienten angewandt, die sich in einem kritischen Zustand befinden. Dabei kann es erforderlich werden, dass kurzfristig ein Defibrillator eingesetzt wird, ohne dass genügend Zeit besteht, Diagnosegeräte regulär vom Patienten zu trennen. Dadurch besteht die Gefahr einer Schädigung der Diagnosegeräte durch Überspannungsimpulse.

Die Defibrillation ist bei lebensbedrohlichen Situationen wie Kammerflimmern oder pulsloser Kammertachykardie die einzige effektive und lebenserhaltende Maßnahme.

Jede Verzögerung, die sich durch das Abnehmen von Elektroden oder elektrischen Anschlüssen vom Patienten ergeben würde, ist zu vermeiden.

Gemäß dem Stand der Technik verwendet man bei reinen EKG-Geräten oder bei kombinierten EKG-Impedanzmessgeräten, die nicht für bildgebende Verfahren verwendet werden, Eingangswiderstände im Bereich von 10-50 kOhm, um technische Schäden durch den Einsatz von Defibrillatoren zu verhindern.

Die besondere Schwierigkeit bei impedanztomographischen Verfahren besteht darin, dass bei Anwendungen in der thorakalen elektrischen Impedanztomographie im Gegensatz zur reinen Elektrokardiographie die Elektroden häufig für einen doppelten Einsatzzweck vorgesehen sind.

Sie sollen erstens die Anregungsströme, die bis zu 10 mA betragen können und mit denen eine gut auswertbare Potentialverteilung im Patienten erreicht werden soll, in den Patienten einleiten.

Zweitens sollen sie die geringen Signalspannungen, die aufgrund der mit den Anregungsströmen erzeugten Potentialverteilung an der Hautoberfläche des Patienten gemessen werden, dem Eingangsverstärker oder einer Auswerteeinheit wieder zuführen. Die zu messenden Signalspannungen können im Mikrovolt-Volt bis Millivolt-Bereich liegen.

Die einzuleitenden Ströme müssen mit bis zu 10 mA hoch gewählt werden, um ausreichende Potentialunterschiede im gesamten Thorax zu erzeugen, um aus den abgegriffenen Potentialen ein Bild erzeugen zu können. Über Widerständen von 10-50 kOhm würden dabei Spannungen von 100-500 V abfallen. Solche Spannungen können am Patienten nicht zum Einsatz gebracht werden, weshalb die Möglichkeit der Absicherung des Impedanztomographen durch ausreichend hohe Schutzwiderstände nicht in Betracht zu ziehen ist.

Eine Absicherung der Eingänge durch parallel zum Eingangsverstärker geschaltete Varistoren oder Dioden ist ebenfalls problematisch. Zusätzliche Streukapazitäten, die zwischen Signalleitung und Bezugspotential geschaltet werden, müssen möglichst gering gehalten werden. Das ist erforderlich, damit bei den üblichen Arbeitsfrequenzen von etwa 10-200 kHz keine inakzeptablen Blindwiderstände entstehen, die parallel zum Eingang der ersten Verstärkerstufe liegen. Sie würden die Last unzulässig erhöhen, die der Messkreis gegenüber der Potentialverteilung auf der Hautoberfläche des Patienten darstellt und somit die Messung verfälschen. Bei einer Frequenz von 50 kHz stellen 10 pF bereits eine Impedanz von etwa 30 kOhm dar. Damit sind Lösungen nachteilig, die Varistoren oder Dioden parallel zum Eingangsverstärker beinhalten, wenn deren Störkapazität höher als einige pF ist. Dadurch scheiden aber alle Typen aus, die die Ströme, die üblicherweise durch einen Defibrillator-Schlag entstehen, dissipieren könnten.

Es muss daher davon ausgegangen werden, dass der Defibrillator zum Einsatz kommt, ohne dass der Patient vom Impedanztomographen dekonnektiert wird. Neben dem erforderlichen Schutz des Impedanztomographen vor einer Überlastung ergibt sich die Anforderung, eine zu starke Ableitung der Energie des Defibrillatorpulses zu vermeiden, um die Wirksamkeit des Defibrillators nicht unzulässig einzuschränken.

Beispielhaft fordern Normwerke, dass maximal 10% der Energie eines Defibrillatorpulses vom Messkreis dissipiert werden dürfen, wenn noch von einer ausreichenden Wirksamkeit ausgegangen werden soll.

Äquivalente normative Forderungen für thorakale Impedanztomographen sind im Falle einer Etablierung dieses diagnostischen Verfahrens zweifellos zu erwarten. Vor dem Schutz eventuell gefährdeter Gerätekomponenten steht die Gewährleistung eines wirkungsvollen Einsatzes des Defibrillators zum Schutze des Patienten.

Es bestand deshalb die Anforderung eine Vorrichtung bereitzustellen, die einen an einen Patienten angeschlossenen thorakalen elektrischen Impedanztomographen im Fall der Anwendung eines Defibrillators sicher vor Überspannungsschäden schützt, ohne dass die Gefahr besteht, dass die Energie der Defibrillatorpulse in einem Maße abgeleitet wird, die der Wirksamkeit der Defibrillation abträglich ist, wobei die Funktionsfähigkeit des Impedanztomographen erhalten bleiben soll.

Eine Vorrichtung zum Schutz eines elektrischen Impedanztomographen, die die zuvor genannte Anforderung erfüllt, ist beispielsweise aus der Druckschrift DE 10 2005 041 385 A1 bekannt. Offenbart wird darin ein elektrischer Impedanztomograph, dessen Signaleingänge mit einer Schutzschaltung versehen sind, die die Signaleingänge bei einem Anliegen einer für den normalen Messbetrieb zu hohen Spannung gegen zu hohe Eingangsströme sichert, wobei die Schutzschaltung in einem mit dem Impedanztomographen verwendeten Elektrodenträger integriert ist.

Aus der US 2013/0345535 A1 und der US 4 744 369 A1 sind Vorrichtungen zum Verbinden von EKG_Geräten mit Elektroden bekannt, wobei die Vorrichtungen Schutzschaltungen aufweisen.

In der Praxis werden für gewöhnlich Elektrodenträger mit einer Mehrzahl von Hautelektroden verwendet, die in einer Längsrichtung des Elektrodenträgers voneinander beabstandet an dem Elektrodenträger angeordnet sind. So können beispielsweise mindestens zwei, drei, fünf, zehn, fünfzehn oder mehr Hautelektroden vorgesehen sein. Wird nun die aus der zuvor genannten Druckschrift verwendete Vorrichtung zum Schutz des elektrischen Impedanztomographen verwendet, sind sämtliche Hautelektroden zunächst elektrisch mit der Schutzschaltung zu verbinden, um den in Signalleitungsrichtung nachgeordneten, elektrischen Impedanztomographen sicher vor Überspannungsschäden zu schützen. Die elektrischen Verbindungen zwischen der bevorzugt in den Elektrodenträger integrierten Schutzschaltung und der Vielzahl von Hautelektroden sind jeweils besonders elektrisch geschirmt, um zu verhindern, dass der Anwender den zuvor genannten hohen Spannungen, von etwa 100 Volt bis 500 Volt, ungewollt ausgesetzt ist. Mit anderen Worten sind die Hautelektroden durch besonders ausgebildete elektrische Leitungsverbindungen sternförmig mit der einen in den Elektrodenträger integrierten Schutzschaltung zu verbinden.

Bei der zuvor genannten sternförmigen Verbindungsart hat sich herausgestellt, dass entsprechende elektrische Leitungen oft sehr aufwendig und deshalb sehr kostenintensiv herzustellen sind. Denn neben dem vorzusehenden Schutz müssen derartigen Verbindungen auch eine hohe Biegsamkeit bieten, damit sich der Elektrodenträger möglichst abstandslos an den Körper des Patienten anlegen lässt.

Der Erfindung liegt deshalb die Aufgabe zugrunde, einen kostengünstigen und einfach herstellbaren Überspannungsschutz für einen Verbund aus Impedanztomographen und einem biegsamen Elektrodenträger und mehreren Hautelektroden bereitzustellen, wobei der Überspannungsschutz den Impedanztomographen im Fall der Anwendung eines Defibrillators sicher vor Überspannungsschäden schützen soll. Insbesondere sollen die zuvor genannten Probleme behoben werden.

Gemäß einem ersten Aspekt wird die Aufgabe gelöst durch ein System gemäß Anspruch 1.

Vorgesehen ist dazu ein System mit einer Verbindungseinheit mit deren Hilfe ein Impedanztomograph mit einer ersten und einer zweiten Hautelektroden eines des Systems elektrisch verbindbar ist, wobei die Hautelektroden in Längsrichtung des Elektrodenträgers voneinander beabstandet an dem Elektrodenträger angeordnet sind, eine ersten Gegenkontaktvorrichtung an dem Elektrodenträger angeordnet und mit der ersten Hautelektrode verbunden ist, eine zweiten Gegenkontaktvorrichtung an dem Elektrodenträger angeordnet und mit der zweiten Hautelektrode verbunden ist, und wobei die Verbindungseinheit eine erste und eine zweite Kontaktvorrichtung aufweist, die erste Kontaktvorrichtung mit der ersten Gegenkontaktvorrichtung lösbar verbindbar ausgebildet ist, die zweite Kontaktvorrichtung mit der zweiten Gegenkontaktvorrichtung lösbar verbindbar ausgebildet ist, und die erste und die zweite Kontaktvorrichtung jeweils eine elektrische Schutzschaltung aufweisen. Bei den Schutzschaltungen handelt es sich vorzugsweise um Überspannungsschutzschaltungen. Grundsätzlich könnten die Schutzschaltungen derart ausgestaltet sein, um vorbestimmte Signale, insbesondere ab einem vorbestimmten Amplitudenanstieg, ab einer bestimmten Frequenz, ab einer bestimmten Spannung und/oder ab einem bestimmten Strom zu blockieren und/oder zu dämpfen.

Der Erfindung liegt die Erkenntnis zugrunde, dass eine zentrale Schutzschaltung einen verhältnismäßig großen Bauraum einnimmt, so dass bei einer Integration einer derartigen Schutzschaltung in den Elektrodenträger dieser seine notwendige Biegsamkeit verliert, um lückenlos an den Körper eines Patienten angelegt werden zu können. Darüber hinaus wurde festgellt, dass die elektrischen Verbindungsleitungen vorzugsweise sternförmig zu den Hautelektroden ausgebildet sind. Diese Verbindungsleitungen sollten sowohl flexibel verformbar als auch elektrisch abschirmend und geschützt ausgebildet sein. Derartige elektrische Verbindungsleitungen sind nur mit einem sehr hohen Kostenaufwand herstellbar. Der Erfindung liegt deshalb der Gedanke zugrunde, mehrere Schutzschaltungen, insbesondere zwei Schutzschaltungen, vorzusehen, die jeweils mit unterschiedlichen Hautelektroden verbindbar sind. Die Hautelektroden sind an dem Elektrodenträger angeordnet. Zur elektrischen Kontaktierung sind für den Elektrodenträger außerdem Gegenkontaktelemente vorgesehen, um die Hautelektroden elektrisch an den Impedanztomographen anzuschließen. Erfindungsgemäß ist es nun vorgesehen, dass zumindest die Gegenkontaktelemente, die mit der ersten Hautelektrode bzw. der zweiten Hautelektrode elektrisch verbunden sind, jeweils mit einer erfindungsgemäßen Kontaktvorrichtung verbindbar sind, wobei die Kontaktvorrichtungen jeweils eine Schutzschaltung aufweisen. Mit einer Schutzschaltung ist vorzugsweise eine Überspannungsschutzschaltung gemeint. Damit kann gewährleistet werden, dass die Schutzschaltungen elektrodennah angeordnet werden können. Die elektrischen Verbindungen zwischen den Schutzschaltungen und der jeweils mindestens einen koppelbaren Hautelektrode kann sehr kurz ausgestaltet werden. Denn die entsprechende Verbindung erstreckt sich zumindest im Wesentlichen zwischen der gekoppelten Hautelektrode und dem verbundenen Gegenkontaktelement. Die Verbindung ist also sehr kurz und damit günstig herzustellen. Darüber hinaus sind die erfindungsgemäßen Schutzschaltungen jeweils mit einer Hautelektrode elektrisch gekoppelt oder nur mit wenigen Hautelektroden elektrisch gekoppelt, so beispielsweise maximal zwei, drei oder vier Hautelektroden. Deshalb können die Schutzschaltungen jeweils sehr kompakt ausgestaltet sein. Zum elektrischen Kontaktieren sind die Gegenkontaktelemente außenseitig an dem Elektrodenträger angeordnet. Werden die Kontaktvorrichtungen jeweils mit einer der Gegenkontaktelemente elektrisch verbunden, können die Kontaktvorrichtungen an dem jeweiligen Gegenkontaktelement auch befestigt werden. Jede der Kontaktvorrichtungen weist deshalb vorzugsweise einen Anschluss auf, der mechanisch mit der Gegenkontaktvorrichtung verbindbar ist. Besonders bevorzugt bildet sich zwischen jeder der Kontaktvorrichtungen und der zugehörigen Gegenkontaktvorrichtung eine Steckverbindung aus. Alternativ kann eine Befestigung an dem Elektrodenträger erfolgen. Durch ihre kompakte Bauform behindern die Kontaktvorrichtungen den Elektrodenträger jedoch nicht in seiner Biegsamkeit. Denn analog zu den Hautelektroden sind die Gegenkontaktelemente vorzugsweise in Längsrichtung des Elektrodenträger voneinander beabstandet.

Eine vorteilhafte Ausgestaltung der Verbindungeinheit zeichnet sich durch eine elektrische Leitungsverbindung aus, an deren einem, insbesondere mehrteiligen, Ende die erste und zweite Kontaktvorrichtung angeordnet sind und deren anderes Ende mit dem Impedanztomographen verbindbar ausgebildet ist. An dem mit dem Impedanztomographen verbindbaren Ende der Leitungsverbindung können entsprechende Anschlüsse vorgesehen sein. Das andere Ende der Leitungsverbindung kann mehrteilig sein. Gemeint ist damit, dass sich der entsprechende Endabschnitt der Leitungsverbindung in mehrere, elektrische Leitungen aufteilt. Am Ende dieser Leitungen sind dann die Kontaktvorrichtungen angeordnet. Vorzugsweise führen die Leitungen von ihrem jeweiligen Ende an den Kontaktvorrichtungen sternförmig in dem zuvor genannten Endabschnitt der Leitungsverbindung zusammen. Wie zuvor erläutert, dienen die Kontaktvorrichtungen zum Verbinden der Hauptelektroden bzw. der jeweiligen zugehörigen Gegenkontaktvorrichtungen mit der elektrischen Leitungsverbindung beziehungsweise mit dem Impedanztomographen. Mit einer Leitungsverbindung, deren eines Ende mehrteilig zum Anordnen für die Kontaktvorrichtungen ausgebildet ist, kann die Anzahl ein Leitungsverbindungen zum Anschluss von Hautelektroden an einen Impedanztomographen minimiert werden. Vorzugsweise ist nur eine Leitungsverbindung zwischen dem Impedanztomographen und den Kontaktvorrichtungen bzw. Hauptelektroden notwendig.

Eine weitere vorteilhafte Ausgestaltung der Verbindungseinheit zeichnet sich dadurch aus, dass die elektrische Leitungsverbindung und die Kontaktvorrichtungen eine Einheit bilden. Die elektrische Leitungsverbindung und die zumindest beiden Kontaktvorrichtungen bilden somit eine gemeinsame Einheit, wobei ihre jeweiligen funktionalen Abschnitte aneinander grenzen. Ihre Funktionen, also bei der Leitungsverbindung das Weiterleiten von elektrischen Signalen und bei der Schutzschaltung das Blockieren und/oder Dämpfen von bestimmten Signalarten, bleiben weiterhin getrennt. Damit vereinfacht sich die Kontaktherstellung zwischen dem Impedanztomographen und den Hautelektroden. Denn die zuvor genannte Einheit kann von einer, einen entsprechenden Aufbau installierenden Person besonders einfach und schnell verwendet werden. Außerdem verringerte sich eine Gefahr einer fehlerhaften Installation der Leitungsverbindung und der Kontaktvorrichtungen.

Eine weitere vorteilhafte Ausgestaltung der Verbindungseinheit zeichnet sich dadurch aus, dass die Schutzschaltungen jeweils einen Kondensator und/oder passive Bauteile aufweisen. Die Schutzschaltungen sind elektrische Schutzschaltungen. Mit ihnen soll also die Weiterleitung von vorbestimmten elektrischen Signalen verhindert und/oder in der Weise geändert, insbesondere gedämpft, werden, bevor die Signale nachfolgenden Geräte und/oder elektrische Einheiten gefährdet. Schutzschaltungen mit jeweils einem Kondensator haben sich als vorteilhaft erwiesen, um hohe Spannungen an der Weiterleitung zu hindern. Die Schutzschaltungen sind deshalb vorzugsweise Überspannung-Schutzschaltungen. Weitere passive Bauteile haben sich ebenfalls als vorteilhaft erwiesen, da diese sehr robust sind. Sie eignen sich deshalb für einen Einsatz mit einer langen Lebensdauer.

Eine weitere vorteilhafte Ausgestaltung der Verbindungseinheit zeichnet sich dadurch aus, dass die Schutzschaltungen jeweils eine elektrische Abschirmung aufweisen. Mit der elektrischen Abstimmung wird verhindert, dass die Schutzschaltungen von äußeren elektrischen Potenzialen in ihrer vorgesehen Funktionsweise negativ beeinflusst werden. Damit wird die Betriebssicherheit der Schutzschaltungen besonders einfach und effektiv gewährleistet.

Das erfindungsgemäße System umfasst außerdem eine erste Gegenkontaktvorrichtung, die an dem Elektrodenträger angeordnet und mit der ersten Hautelektrode verbunden ist, und eine zweite Gegenkontaktvorrichtung, die an dem Elektrodenträger angeordnet und mit der zweiten Hautelektrode verbunden ist, wobei die erste Kontaktvorrichtung der Verbindungseinheit mit der ersten Gegenkontaktvorrichtung lösbar verbunden ist, und die zweite Kontaktvorrichtung mit der zweiten Gegenkontaktvorrichtung lösbar verbunden ist.

Dabei gelten Merkmale, Details und Vorteile, die im Zusammenhang mit dem erfindungsgemäßen Verbindungseinheit beschrieben sind, selbstverständlich auch im Zusammenhang mit dem erfindungsgemäßen System und jeweils umgekehrt, so dass bezüglich der Offenbarung zu den einzelnen Erfindungsaspekten stets wechselseitig Bezug genommen wird beziehungsweise werden kann.

Eine vorteilhafte Ausgestaltung des Systems zeichnet sich dadurch aus, dass der Impedanztomograph dem System zugeordnet ist, wobei der Impedanztomograph mittels der elektrischen Leitungsverbindung mit der ersten und der zweiten Hautelektrode elektrisch verbunden ist. Besonders bevorzugt ist der elektrischen Leitungsverbindung zumindest die erste und die zweite Kontaktvorrichtung zugeordnet. Somit kann die Leitungsverbindung zu einem Ende an dem Impedanztomographen angeschlossen werden und mit dem anderen Ende, an dem die Kontaktvorrichtungen angeordnet sind, mit den Gegenkontaktvorrichtungen verbunden werden, um die Hautelektroden mit dem Impedanztomographen elektrisch zu verbinden. Mit den elektrischen Schutzschaltungen, die von den Kontaktvorrichtungen umfasst sind, wird der Impedanztomographen geschützt. Insbesondere wird verhindert, dass der Impedanztomograph einer zu hohen Spannung, insbesondere hervorgerufenen durch einen Defibrillator, ausgesetzt wird.

Das erfindungsgemäße System zeichnet sich dadurch aus, dass die Schutzschaltungen jeweils ein Teil einer mehrteiligen Schutzschaltungseinheit sind, wobei weitere Teile der Schutzschaltungseinheit in den Elektrodenträger, in die elektrische Leitungsverbindung, in die Hautelektroden und/oder den Impedanztomographen integriert sind. Mit den Schutzschaltungen sind die Schutzschaltungen der Kontaktvorrichtungen gemeint. Die Schutzschaltungen sind dabei vorzugsweise als Überspannungsschutzschaltungen ausgebildet. Mit einer derartigen Überspannungsschutzschaltung werden vorzugsweise bestimmte Spannungen, die eine bestimmte Amplitude überschreiten, an der Weiterleitung behindert. Ist es vorgesehen, dass auch andere Signalarten an einer Weiterleitung gehindert oder vor ihrer Weiterleitung verändert werden, können weitere Bauteile vorgesehen werden. So ist es beispielsweise denkbar, dass nur Frequenzen aus einem vorbestimmten Frequenzspektrum an dem Impedanztomographen weitergeleitet werden sollen, um den Impedanztomographen vor Signalen mit einer Frequenz außerhalb des vorbestimmten Frequenzspektrums zu schützen. Sodann wäre eine entsprechende Filterung vorsehbar. Diese könnte einen weiteren Teil der Schutzschaltungseinheit bilden. Sie wäre deshalb beispielsweise in die elektrische Leitungsverbindung integrierbar. Der Teil einer jeden Schutzschaltungseinheit, der vor Überspannungen schützt, soll aber vorzugsweise von der entsprechenden Kontaktvorrichtung umfasst sein.

Weitere vorteilhafte Merkmale der Erfindung werden aus der Beschreibung der erfindungsgemäßen Ausführungsformen zusammen mit den Ansprüchen und/oder den beigefügten Zeichnung ersichtlich. Dabei können erfindungsgemäße Ausführungsformen einzelne oder eine Kombination mehrerer Merkmale erfüllen. Die Erfindung wird nachstehend ohne Beschränkung des allgemeinen Erfindungsgedankens anhand von einem Ausführungsbeispiel unter Bezugnahme die Zeichnung beschrieben. In den Zeichnung zeigt:

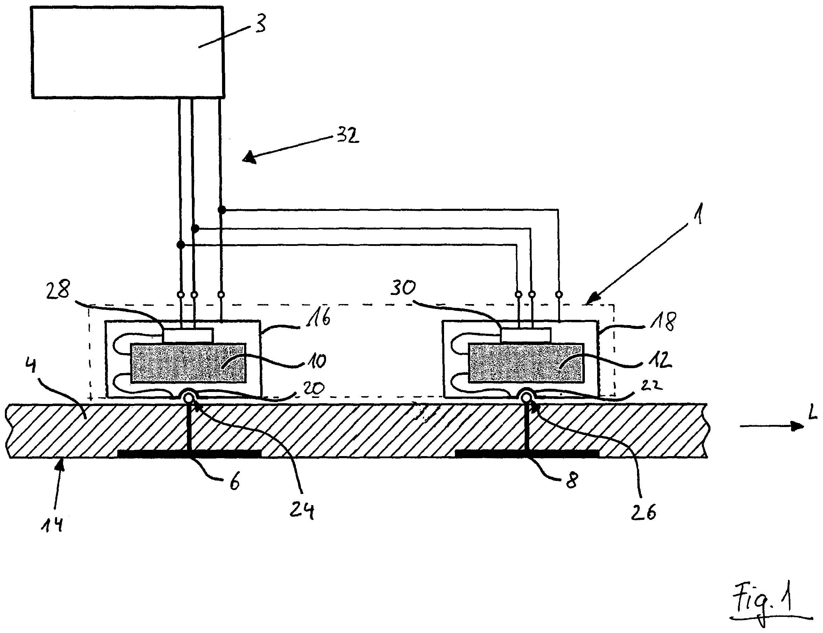

- Figur 1: eine schematische Schnittdarstellung der erfindungsgemäßen Verbindungseinheit.

In der Figur 1 ist die erfindungsgemäße Verbindungseinheit 1 (gestrichelter Kasten) für einen elektrischen Impedanztomographen 3 schematisch gezeigt. Die Verbindungseinheit 1 umfasst eine erste Kontaktvorrichtung 16 und eine zweite Kontaktvorrichtung 18. Die Kontaktvorrichtungen 16, 18 können getrennt voneinander ausgebildet sein. Alternativ kann es vorgesehen sein, dass die Kontaktvorrichtung im 16, 18 mechanisch miteinander verbunden bzw. gekoppelt sind.

Aus der Figur 1 ist außerdem ein Elektrodenträger 4 erkennbar. Hierbei handelt es sich vorzugsweise um einen sog. Elektrodengürtel. Der Elektrodenträger 4 kann also wie ein Gürtel um den Körper eines Patienten gelegt werden. Um die für die Impedanztomographie notwendigen elektrische Signale an den Körper zu übermitteln oder elektrische Signale aufzunehmen, sind die erste und die zweite Hautelektrode 6, 8 in der Längsrichtung L des Elektrodenträgers 4 voneinander beabstandet an dem Elektrodenträger 4 angeordnet. Wird der Elektrodenträger 4 ringförmig gebogen, so sind die Hautelektroden 6, 8 radial innenseitig angeordnet. Die Hautelektroden 6, 8 kommen also unmittelbar mit dem Körper des Patienten in Kontakt und können somit die Übermittelung der gewünschten Signale gewährleisten. Wie aus der Figur 1 hervorgeht, können die Hautelektroden 6, 8 stetig mit einer Stirnfläche 14 des Elektrodenträgers 4 abschließen. Sie bilden sodann eine gemeinsame Oberfläche des Elektrodenträgers 4 bzw. der Vorrichtung 2.

Zu der von der Stirnseite 14 des Elektrodenträgers 4 abgewandten Seiten sind die Hautelektrode 6, 8 jeweils mit einer Gegenkontaktvorrichtung 24, 26 verbunden. So ist die erste Hautelektrode 6 mit der ersten Gegenkontaktvorrichtung 24 verbunden, die an der Rückseite des Elektrodenträgers 4 angeordnet ist. Die zweite Hautelektrode 8 ist mit der zweiten Gegenkontaktvorrichtung 26 verbunden, die ebenfalls an der Rückseite des Elektrodenträgers 4 angeordnet ist. Dabei sind die beiden Gegenkontaktvorrichtungen jeweils als Anschlusselement ausgebildet und ragen jeweils über den Elektrodenträger 4 hinaus. Die beiden Gegenkontaktvorrichtungen 24, 26 sind in Längsrichtung L des Elektrodenträger 4 voneinander beanstandet. Mit den Gegenkontaktvorrichtungen 24, 26 sind die Kontaktvorrichtungen 16, 18 elektrisch und mechanisch lösbar verbindbar. Die Kontaktvorrichtungen 16, 18 können an den Gegenkontaktvorrichtung 24, 26 auch vollständig mechanisch befestigt sein.

In einer für einen Patienten kritischen Situation kann es vorkommen, dass der Impedanztomograph 3 und ein Defibrillator zeitgleich zur Anwendung kommen. Von dem Defibrillator werden hohe Spannungsimpulse ausgesendet. Um zu verhindern, dass die vom Defibrillator ausgesendeten Spannungsimpulse den Impedanztomographen 3 schädigen oder sogar zerstören, ist es erfindungsgemäßen vorgesehen, dass die erste Kontaktvorrichtung 16 eine elektrische Schutzschaltung 10 und die zweite Kontaktvorrichtung 18 eine elektrische Schutzschaltung 12 aufweisen. Ein Spannungsimpuls des Defibrillator, der von einer Hautelektrode 6, 8 an die zugehörigen Kontaktvorrichtung 16, 18 geleitet wird, trifft sodann auf die entsprechende Schutzschaltung 10, 12 die eine Weiterleitung des hohen Spannungsimpulses verhindert. Der Impedanztomograph 3 ist also vor derartigen Spannungsimpulsen effektiv geschützt.

Um eine elektrische Verbindung zwischen den Kontaktvorrichtungen 16, 18 und dem Impedanztomographen 3 herzustellen, ist eine elektrische Leitungsverbindung 32, also vorzugsweise ein Bündel aus elektrischen Leitungen, vorgesehen. Die Leitungsverbindung 32 erstreckt sich von einem Anschluss des Impedanztomographen 3 zu der ersten Gegenkontaktvorrichtung 16 und der zweiten Gegenkontaktvorrichtung 18. Ein Ende der elektrischen Leitungsverbindung 32 ist deshalb vorzugsweise mehrteilig ausgebildet. Die elektrische Leitungsverbindung 32 weist also einen Endabschnitt auf, der sich in mehreren elektrische Leitungen aufgeteilt.

Für eine einfache Handhabung der elektrischen Leitungsverbindung 32 und der Kontaktvorrichtungen 16, 18 bilden die elektrische Leitungsverbindung und die Kontaktvorrichtungen 16, 18 eine Einheit. Um nun den Defibrillator 3 mit den Hautelektroden 6, 8 zu verbinden, werden die zuvor genannte Einheit einem Ende mit dem Impedanztomographen 3 und mit dem anderen Ende, an dem die erste Kontaktvorrichtung 16 und die zweite Kontaktvorrichtungen 18 angeordnet sind, mit der ersten Gegenkontaktvorrichtung 24 und der zweiten Gegenkontaktvorrichtung 26 verbunden. Zwischen der ersten Kontaktvorrichtung 16 und der ersten Gegenkontaktvorrichtung 24 bildet sich vorzugsweise eine Schnellverbindung, insbesondere eine Steckverbindung, aus. So kann die Gegenkontaktvorrichtung 16 eine erste Aufnahme 20 aufweisen, in die die Gegenkontaktvorrichtung 24 einsteckbar ist, um sodann die mechanische und/oder elektrische Verbindung zwischen der ersten Kontaktvorrichtung 16 und der erste Gegenkontaktvorrichtung 24 herzustellen. Die Steckverbindung kann auch umgekehrt ausgebildet sein. Analog zu der ersten Kontaktvorrichtung 16 weist auch die zweite Kontaktvorrichtung 18 eine zweite Aufnahme 22 auf, in die die zweite Gegenkontaktvorrichtung 26 einsteckbar ist, um sodann die mechanische und/oder elektrische Verbindung zwischen der zweiten Kontaktvorrichtung 18 und der zweiten Gegenkontaktvorrichtung 26 herzustellen. Auch hier ist eine umgekehrte Steckverbindung möglich.

Aus der Figur 1 ist außerdem zu erkennen, dass die erste Aufnahme 20 der ersten Kontaktvorrichtung 16 elektrisch mit der ersten Schutzschaltung 10 und die ersten Schutzschaltung 10 wiederum mit einer ersten Vorverarbeitungseinheit 28 verbunden ist. Die erste Vorverarbeitungseinheit 28 ist dabei vorzugsweise der ersten Kontaktvorrichtung 16 zugeordnet. Die erste Aufnahme 20, die erste Schutzschaltung 10 und die erste Vorverarbeitungseinheit 28 sind also elektrisch in Reihe geschaltet und vorzugsweise von der ersten Kontaktvorrichtung 16 umfasst. Die Vorverarbeitungseinheit 28 kann als eine Verarbeitungseinheit von elektrischen Signalen, beispielsweise als eine Datenverarbeitungseinheit, als Messauswerteeinheit oder dergleichen, ausgebildet sein. Alternativ kann die Vorverarbeitungseinheit 28 als eine Signalverstärkungseinheit ausgebildet sein. Für beide Alternativen weist die Vorverarbeitungseinheit 28 aktive, elektrische Bauelemente auf. Aufgrund der zuvor genannten Reihenschaltung schützt die erste Schutzschaltung 10 bereits die aktiven Bauelemente der ersten Kontaktvorrichtung 16. An die erste Vorverarbeitungseinheit 28 schließt die elektrische Leitungsverbindung 32 an, um die erste Kontaktvorrichtung 16 mit dem Impedanztomographen 3 zu verbinden. Wie bereits die erste Vorverarbeitungseinheit 28 ist auch der Impedanztomograph 3 mit seinen aktiven, elektrischen Komponenten durch die erste Schutzschaltung 10 geschützt.

Die zweite Kontaktvorrichtung 18 ist analog zu der ersten Kontaktvorrichtung 16 aufgebaut und/oder ausgebildet. Auf die vorherigen Ausführungen wird deshalb in analoger Weise für die zweite Kontaktvorrichtung 18 Bezug genommen. So weist die zweite Kontaktvorrichtung 18 beispielsweise eine Reihenschaltung von einer zweiten Aufnahme 22, der zweiten Schutzschaltung 12 und einer zweiten Vorverarbeitungseinheit 30 auf. An die zweite Vorverarbeitungseinheit 30 schließt die elektrische Leitungsverbindung 32 zu dem Impedanztomographen 3. Es gelten deshalb die analogen Effekte und/oder Vorteile.

### BEZUGSZEICHENLISTE

### (Teil der Beschreibung)

- L: Längsrichtung
- 1: Verbindungseinheit
- 3: Impedanztomograph
- 4: Elektrodenträger
- 6: erste Hautelektrode
- 8: zweite Hautelektrode
- 10: erste Schutzschaltung
- 12: zweite Schutzschaltung
- 14: Stirnfläche
- 16: erste Kontaktvorrichtung
- 18: zweite Kontaktvorrichtung
- 20: erste Aufnahme
- 22: zweite Aufnahme
- 24: erste Gegenkontaktvorrichtung
- 26: erste Gegenkontaktvorrichtung
- 28: erste Vorverarbeitungseinheit
- 30: zweite Vorverarbeitungseinheit
- 32: elektrische Leitungsverbindung

## Patentansprüche

1. System für einen elektrischen Impedanztomographen (3) mit
- einem Elektrodenträger (4),
- einer ersten und einer zweiten Hautelektrode (6, 8), die in Längsrichtung L des Elektrodenträgers (4) voneinander beabstandet an dem Elektrodenträger (4) angeordnet sind,
- einer ersten Gegenkontaktvorrichtung (24), die an dem Elektrodenträger (4) angeordnet und mit der ersten Hautelektrode (6) verbunden ist, und
- einer zweiten Gegenkontaktvorrichtung, die an dem Elektrodenträger (4) angeordnet und mit der zweiten Hautelektrode (8) verbunden ist, wobei
- das System eine Verbindungseinheit (1) mit deren Hilfe ein Impedanztomograph (3) mit einer ersten und einer zweiten Hautelektrode (6, 8) eines Elektrodenträgers (4) elektrisch verbindbar ist, aufweist, wobei die Verbindungseinheit (1) eine erste und eine zweite Kontaktvorrichtung (16, 18) aufweist, wobei die erste und die zweite Kontaktvorrichtung (16, 18) jeweils eine elektrische Schutzschaltung (10, 12) aufweisen, wobei
- die erste Kontaktvorrichtung (16) mit der ersten Gegenkontaktvorrichtung (24) lösbar verbunden ist, und
- die zweite Kontaktvorrichtung (18) mit der zweiten Gegenkontaktvorrichtung (26) lösbar verbunden ist, wobei die Schutzschaltungen (10, 12) jeweils ein Teil einer mehrteiligen Schutzschaltungseinheit sind, wobei weitere Teile der Schutzschaltungseinheit in den Elektrodenträger (4) und/oder in die Hautelektroden (6, 8) integriert sind.

2. System nach dem vorhergehenden Anspruch, **gekennzeichnet durch** eine elektrische Leitungsverbindung (32), an deren einem, insbesondere mehrteiligen, Ende die erste und zweite Kontaktvorrichtung (16, 18) angeordnet sind und deren anderes Ende mit dem Impedanztomographen (3) verbindbar ausgebildet ist.

3. System nach dem vorhergehenden Anspruch, **dadurch gekennzeichnet, dass** die elektrische Leitungsverbindung (32) und die Kontaktvorrichtungen (16, 18) eine Einheit bilden.

4. System nach einem der vorhergehenden Ansprüche, **dadurch gekennzeichnet, dass** die Schutzschaltungen (10, 12) jeweils einen Kondensator und/oder passive Bauteile aufweisen.

5. System nach einem der vorhergehenden Ansprüche, **dadurch gekennzeichnet, dass** die Schutzschaltungen (10, 12) jeweils eine elektrische Abschirmung aufweisen.

6. System nach einem der vorhergehenden Ansprüche, **dadurch gekennzeichnet, dass** der Impedanztomograph (3) dem System zugeordnet ist, wobei der Impedanztomograph (3) mittels der elektrischen Leitungsverbindung (32) mit der ersten und der zweiten Hautelektrode (6, 8) elektrisch verbunden ist.

## Claims

1. System for an electrical impedance tomograph (3) comprising
- an electrode carrier (4),
- a first and a second skin electrode (6, 8), which are arranged on the electrode carrier (4), spaced apart from one another along the longitudinal direction L of the electrode carrier (4),
- a first mating contact apparatus (24), which is arranged on the electrode carrier (4) and connected to the first skin electrode (6), and
- a second mating contact apparatus, which is arranged on the electrode carrier (4) and connected to the second skin electrode (8), wherein
- the system has a connection unit (1), with the aid of which an impedance tomograph (3) is electrically connectable to a first and a second skin electrode (6, 8) of an electrode carrier (4), wherein the connection unit (1) has a first and a second contact apparatus (16, 18), wherein the first and the second contact apparatus (16, 18) each have an electrical protective circuit (10, 12), wherein
- the first contact apparatus (16) is detachably connected to the first mating contact apparatus (24), and
- the second contact apparatus (18) is detachably connected to the second mating contact apparatus (26), wherein the protective circuits (10, 12) each are a part of a multi-part protective circuit unit, with further parts of the protective circuit unit being integrated in the electrode carrier (4) and/or in the skin electrodes (6, 8).

2. System according to the preceding claim, **characterized by** an electrical wired connection (32), the first and second contact apparatus (16, 18) being arranged at an, in particular multi-part, end of said electrical wired connection and the other end thereof being embodied to be connectable to the impedance tomograph (3).

3. System according to the preceding claim, **characterized in that** the electrical wired connection (32) and the contact apparatuses (16, 18) form a unit.

4. System according to any one of the preceding claims, **characterized in that** the protective circuits (10, 12) each have a capacitor and/or passive components.

5. System according to any one of the preceding claims, **characterized in that** the protective circuits (10, 12) each have electrical shielding.

6. System according to any one of the preceding claims, **characterized in that** the impedance tomograph (3) is assigned to the system, wherein the impedance tomograph (3) is electrically connected to the first and the second skin electrode (6, 8) by means of the electrical wired connection (32).

## Revendications

1. Système destiné à un tomographe à impédance électrique (3) comportant
- un porte-électrode (4),
- des première et deuxième électrodes cutanées (6, 8) qui sont disposées sur le porte-électrode (4) en étant espacées l'une de l'autre dans le sens longitudinal L du porte-électrode (4),
- un premier dispositif de contact homologue (24) disposé sur le porte-électrode (4) et relié à la première électrode cutanée (6), et
- un deuxième dispositif de contact homologue disposé sur le porte-électrode (4) et relié à la deuxième électrode cutanée (8), dans lequel
- le système comporte une unité de liaison (1) à l'aide de laquelle un tomographe à impédance (3) peut être relié électriquement à des première et deuxième électrodes cutanées (6, 8) d'un porte-électrode (4), dans lequel l'unité de liaison (1) possède des premier et deuxième dispositifs de contact (16, 18), dans lequel les premier et deuxième dispositifs de contact (16, 18) possèdent respectivement un circuit de protection électrique (10, 12), dans lequel
- le premier dispositif de contact (16) est relié de manière amovible au premier dispositif de contact homologue (24), et
- le deuxième dispositif de contact (18) est relié de manière amovible au deuxième dispositif de contact homologue (26), dans lequel les circuits de protection (10, 12) font chacun partie d'une unité de circuit de protection en plusieurs parties, dans lequel d'autres parties de l'unité de circuit de protection sont intégrées dans le porte-électrode (4) et/ou dans les électrodes cutanées (6, 8).

2. Système selon la revendication précédente, **caractérisé par** une liaison par ligne électrique (32), qui est en particulier en plusieurs partie, à une extrémité de laquelle sont disposés les premier et deuxième dispositifs de contact (16, 18) et dont l'autre extrémité est réalisée pour être reliée au tomographe à impédance (3).

3. Système selon la revendication précédente, **caractérisé en ce que** la liaison par ligne électrique (32) et les dispositifs de contact (16, 18) forment une unité.

4. Système selon l'une des revendications précédentes, **caractérisé en ce que** les circuits de protection (10, 12) possèdent respectivement un condensateur et/ou des composants passifs.

5. Système selon l'une des revendications précédentes, **caractérisé en ce que** les circuits de protection (10, 12) possèdent respectivement un blindage électrique.

6. Système selon l'une des revendications précédentes, **caractérisé en ce que** le tomographe à impédance (3) est associé au système, dans lequel le tomographe à impédance (3) est relié électriquement aux première et deuxième électrodes cutanées (6, 8) au moyen de la liaison par ligne électrique (32).
